# EUROPEAN PATENT SPECIFICATION

(11) **EP 3 367 954 B1**
(45) Date of publication and mention of the grant of the patent: **27.05.2020**
(21) Application number: 16860698.6
(22) Date of filing: 26.10.2016
(51) Int. Cl.: A61B 90/50, A61B 90/57, A61B 90/25, A61B 18/00, A61B 18/18

(54) **APPARATUSES FOR SECURING A MEDICAL DEVICE AND RELATED METHODS THEREOF**
VORRICHTUNGEN ZUR SICHERUNG EINER MEDIZINISCHEN VORRICHTUNG UND ZUGEHÖRIGE VERFAHREN DAVON
APPAREILS POUR MAINTENIR UN DISPOSITIF MÉDICAL ET LEURS PROCÉDÉS ASSOCIÉS

(30) Priority: 26.10.2015 US 201562246355 P
(43) Date of publication of application: 05.09.2018
(73) Proprietor: Neuwave Medical, Inc., Madison, WI 53704 (US)
(72) Inventor: BISSING, Jeff, Madison, Wisconsin 53704 (US); THOM, Mark, Madison, Wisconsin 53704 (US); SCHEFELKER, Richard W., Madison, Wisconsin 53704 (US); THIEL, Matthew, Madison, Wisconsin 53704 (US); SCHANING, Matt, Madison, Wisconsin 53704 (US); FERGUSON, J. Scott, Madison, Wisconsin 53704 (US)
(74) Representative: Burton, Nick
(86) International application number: PCT/US2016/058890
(87) International publication number: WO 2017/075068

(56) References cited:
- WO-A1-2012/159088
- US-A- 5 540 649
- US-A- 5 597 146
- US-A- 6 165 163
- US-A1- 2005 109 900
- US-A1- 2011 213 352
- US-A1- 2012 182 134
- US-A1- 2012 316 551
- US-A1- 2013 306 543
- US-A1- 2014 046 174
- US-B1- 6 210 323

## Description

### FIELD OF THE INVENTION

The present invention relates to an apparatus for securing a medical device. In particular, the present invention relates to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a favorable position while conducting a medical procedure with such a medical device (e.g., ablation of peripheral lung tissue).

### BACKGROUND OF THE INVENTION

Generally, medical procedures (e.g., ablation of peripheral lung tissue) involving the use of medical devices (e.g., energy delivery devices) require either a physician or a person assisting the physician to physically hold one or more of such devices in a desired favorable position for extended periods of time.

Apparatuses configured to permit the securing of such medical devices at a desired favorable location are needed.

WO 2012/159088 A1 describes a surgical support system may include a primary telescoping support, secondary telescoping support supported from the primary telescoping support and configured for telescopically mounting a surgical tool thereto, such that telescoping of the primary support repositions the secondary telescoping support; and a positioning joint disposed between the primary and secondary telescoping supports for universally positioning and locking the secondary telescoping support with respecting to the primary telescoping support.

### SUMMARY OF THE INVENTION

Embodiments of the present invention can provide an apparatus for securing a medical device. In particular, embodiments of the present invention relate to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a favorable position while conducting a medical procedure with such a medical device (e.g., ablation of peripheral lung tissue).

According to an aspect of the invention, there is provided an apparatus for securing a medical device, comprising
an elongated main body having a main body proximal end and a main body distal end, wherein the length of the main body is greater than the width of the main body, wherein the length of the main body is expandable or retractable, wherein the main body has a length that is extendable to 2 meters and retractable to 0.2 meters, wherein the main body has a width that is between 0.005 meters and 0.3 meters,
an anchoring region engaged with the main body proximal end, wherein the anchoring region is configured to lock onto an anchorable region, wherein upon locking with an anchorable region the anchoring region and elongated main body are locked into a fixed position,
a medical device attachment region having a medical device attachment region distal end and a medical device attachment region proximal end,
wherein the length of the medical device attachment region proximal end is expandable or retractable,
wherein the medical device attachment region proximal end is elongated such that the length of the medical device attachment region proximal end is greater than the width of the medical device attachment region proximal end,
wherein the medical device attachment region proximal end has a length that is extendable to 2 meters and retractable to 0.5 meters, wherein the medical device attachment region proximal end has a width that is between 0.025 meters and 0.3 meters,
wherein the medical device attachment region distal end is configured to secure a medical device, and
a connection region engaging the main body distal end and the medical device attachment region proximal end, wherein the connection region is adjustable such that the medical device attachment region can lockably project in any direction therefrom.

The radial projection may, for example, be from 0° to 360° and/or the rotational projection may, for example, be from 0° to 360°).

The anchoring region is not limited to a particular manner of locking onto an anchorable region. In some embodiments, the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases with decreased amounts of compression, wherein the amount of applied compression is lockable.

The medical device attachment region distal end is not limited to a particular manner of securing a medical device.

In some embodiments, the medical device attachment region distal end is configured with a torsion based securing mechanism, wherein the torsion based securing mechanism naturally rests in a closed manner (e.g., similar to an alligator clamp or clip). In some embodiments, the torsion based securing mechanism is configured to be opened for purposes of accommodating a medical device, wherein releasing the torsion based securing mechanism from an opened manner naturally results in torsion based closing of the torsion based securing mechanism.

In some embodiments, the medical device attachment region distal end has a medical device attachment region distal end compressible region and a medical device attachment region distal end compressor region, wherein the medical device attachment region distal end compressor region is configured to apply a range of compression to the medical device attachment region distal end compressible region such that the size of the medical device attachment region distal end compressible region decreases with increased amounts of compression and increases with decreased amounts of compression, wherein the amount of applied compression is lockable.

In some embodiments, the medical device attachment region further comprises one or more extensions for securing additional medical devices (e.g., additional energy delivery devices, light emitting objects, any other medical device). Such embodiments are not limited to a particular type or kind of extension for securing additional medical devices. In some embodiments, the extension is elongated with an additional device securing feature. In some embodiments, such an extension can be expanded or retracted to any desirable length.

According to another aspect of the invention, there is provided a method of securing a medical device in a desired position, comprising:
securing an apparatus as provided in Claim 1 to an anchorable region,
securing a medical device with the medical device attachment region distal end, wherein the medical device is an energy delivery device configured to ablate tissue,
and adjusting the apparatus such that the secured medical device is in a desired position, wherein the adjusting comprises adjusting one or more of the following:
   adjusting the projection of the medical device attachment region from the connection region,
   adjusting the length of the medical device attachment region proximal end, and
   adjusting the length of the elongated main body.

The energy delivery device may, for example, be a microwave energy delivery device or a radiofrequency energy delivery device.

A method for conducting a medical procedure with a medical device is disclosed, the method comprising securing such apparatus to an anchorable region, securing a medical device with the medical device attachment region distal end, adjusting the apparatus such that the secured medical device is in a desired position, wherein the adjusting comprises adjusting one or more of the following: adjusting the projection of the medical device attachment region from the connection region, adjusting the length of the medical device attachment region proximal end, and adjusting the length of the elongated main body, and conducting the medical procedure with the medical device. In some embodiments, the medical device is an energy delivery device (e.g., a microwave energy delivery device or a radiofrequency energy delivery device). In some embodiments, the medical procedure is an ablation procedure, and the medical device is an energy delivery device.

According to a further aspect of the invention, there is provided a kit comprising an apparatus of Claim 1 and a system, wherein the system comprises:
a primary catheter, wherein the primary catheter comprises a hollow primary lumen;
a channel catheter, wherein the channel catheter is concentrically positioned within the hollow primary lumen, and wherein the channel catheter comprises a channel lumen;
a steerable navigation catheter, wherein the steerable navigation catheter is configured to fit within the channel lumen, and wherein the steerable navigation catheter comprises a steerable tip and position sensing element; and
an energy delivery device, wherein the energy delivery device is configured to fit within the channel lumen, and wherein the microwave energy delivery device comprises, within the channel lumen, first, second and third conductors therein,
wherein the energy delivery device is a microwave ablation device or a radiofrequency energy delivery device.

In some embodiments, the primary catheter comprises a bronchoscope. In some embodiments, the kit further comprises a processor configured to operate power delivery to the energy delivery device (e.g., microwave energy delivery device or radiofrequency energy delivery device).

In some embodiments, the energy delivery device (e.g., microwave energy delivery device or radiofrequency energy delivery device) is capable of delivering energy through the channel catheter.

In some embodiments, the energy delivery device is a microwave energy delivery device. In some embodiments, the microwave energy delivery device comprises a coolant channel and wherein the system comprises a coolant source in fluid communication with the coolant channel. In some embodiments, channel catheter comprises a braided material that provides flexibility. In some embodiments, the inner conductor is hollow. In some embodiments, the hollow of the inner conductor is in fluid communication with a coolant source. In some embodiments, the space between the inner and outer conductors comprises a dielectric material. In some embodiments, the space between the inner and outer conductors comprises air channels. In some embodiments, the kits further comprise a microwave power supply in electrical communication with the microwave energy delivery device.

Methods are disclosed for placing a microwave energy delivery device at a difficult to reach treatment site comprising providing such a kit, wherein the steerable navigation catheter is within the channel lumen, and the channel catheter is concentrically positioned within the hollow primary lumen; securing the apparatus to an anchorable region; securing the microwave energy delivery device with the medical device attachment region distal end; adjusting the apparatus such that the secured microwave energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body;
inserting the primary catheter into an opening in a subject and directing the primary catheter towards the treatment site until further advance is constrained by the diameter of the primary catheter; advancing the channel catheter beyond the distal end of the primary catheter and extending the channel catheter to the treatment site; securing the distal end of the channel catheter at the treatment site; withdrawing the steerable navigation catheter through the channel lumen and out the proximal end of the channel catheter; and inserting the microwave energy delivery device through the channel lumen until the distal end of the microwave energy delivery device reaches the treatment site.

Such methods are not limited to a particular difficult to reach treatment site. In some embodiments, the difficult to reach treatment site comprises the periphery of the lung. In some embodiments, the difficult to reach treatment site comprises a peripheral lung nodule. In some embodiments, the lung nodule is accessed through the bronchial tree.

Methods are disclosed for treating a peripheral lung tissue region in a subject, comprising providing such a kit, wherein the steerable navigation catheter is within the channel lumen, and the channel catheter is concentrically positioned within the hollow primary lumen; securing the apparatus to an anchorable region; securing the energy delivery device with the medical device attachment region distal end; adjusting the apparatus such that the secured energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; steering the energy delivery device through the subject's lung and positioning the microwave energy delivery device at a target peripheral lung tissue region, and ablating the target peripheral lung tissue region with energy from the microwave energy delivery device, wherein the steering is through the subject's mouth, through the subject's trachea, and through the subject's lung. In some embodiments, the steering comprises advancing the hollow primary catheter having a hollow channel catheter therein through the subject's mouth, through the subject's trachea, and through the subject's lung until further advance is constrained by the diameter of the hollow primary catheter, wherein the hollow channel catheter has therein a steerable navigation catheter; advancing the hollow channel catheter having the steerable navigation catheter therein beyond the distal end of the hollow primary catheter and extending the hollow channel catheter having the steerable navigation catheter therein through the subject's lung and to the target peripheral lung tissue region; withdrawing the steerable navigation catheter from the hollow channel catheter; inserting the energy delivery device through the hollow channel catheter such that it is positioned at the target peripheral lung tissue region.

Advancing the hollow channel catheter having the steerable navigation catheter therein beyond the distal end of the hollow primary catheter and extending the hollow channel catheter having the steerable navigation catheter therein through the subject's lung may comprise extending the hollow channel catheter having the steerable navigation catheter therein through one or more of primary bronchial tissue, secondary bronchial tissue, tertiary bronchial tissue, and bronchiole tissue. The steerable navigation catheter may control the advancing.

The microwave energy delivery device may comprise a braided material. The microwave energy delivery device may be flexible.

Ablating the target peripheral lung tissue region with energy from the microwave energy delivery device may be controlled with a processor.

The microwave energy delivery device may be in electrical communication with an energy power supply. The microwave energy delivery device may comprise one or more coolant channels in fluid communication with a coolant source. The microwave energy delivery device may comprise an inner conductor and an outer conductor.

The inner conductor may be hollow. The inner conductor may be in fluid communication with a coolant source. A dielectric material may be positioned between the inner conductor and the outer conductor. The inner conductor and the outer conductor may comprise air channels.

The target peripheral lung tissue region may comprise lung nodule tissue. The target peripheral lung tissue region may comprise lung tumor tissue. The target peripheral lung tissue region may comprise lung lesion tissue. The target peripheral lung tissue region may comprise cancerous tissue.

One or more stabilization and/or anchoring mechanisms may be used to secure one or more of the hollow primary catheter, the hollow channel catheter, the steerable navigation catheter, and the energy delivery device at a desired tissue region.

The desired tissue region may be the target peripheral lung tissue region.

The microwave energy delivery device may be configured to detect an undesired rise in temperature within the energy delivery device and automatically or manually reduce such an undesired temperature rise through flowing of coolant through the one or more coolant channels. The energy delivery device may be a triaxial microwave probe. The triaxial microwave probe may comprise optimized tuning capabilities to reduce reflective heat loss. The triaxial antenna may comprise an inner conductor, a dielectric material, and an outer conductor, wherein the dielectric material is between the inner conductor and the outer conductor.

### DESCRIPTION OF THE DRAWINGS

FIG. 1 shows a schematic presentation of the apparatus 1. As shown, the apparatus 1 comprises an elongated main body 2, an anchoring region 3, a medical device attachment region 4, and a connection region 5.
FIG. 2 shows drawing of an apparatus 1, having an elongated main body 2, an anchoring region 3, a medical device attachment region 4, and a connection region 5.
FIG. 3 shows a typical mounting procedure with an exemplary apparatus and the securing of a medical device with the apparatus.

### DETAILED DESCRIPTION OF THE INVENTION

The present invention relates to an apparatus for securing a medical device. In particular, the present invention relates to apparatuses for securing medical devices in a desired manner, wherein the apparatuses having an elongated main body, an anchoring region, a medical device attachment region, and a connection region. Such apparatuses are useful for securing a particular medical device (e.g., a microwave energy delivery device) in a desirable position while conducting a medical procedure with such a medical device (e.g., ablation of peripheral lung tissue).

The following discussion includes descriptions of the various embodiments of the apparatus in accordance with the principles of the present disclosure followed by a description of uses of the apparatus.

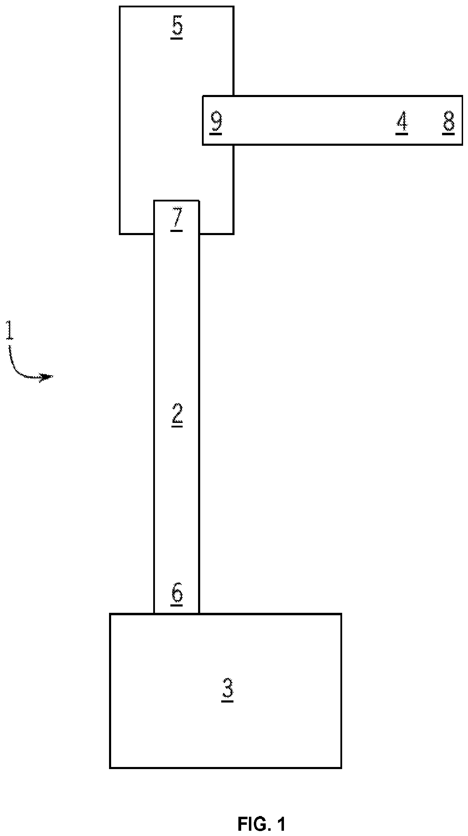

Referring to Figures 1-3, embodiments of the present invention provide apparatuses of the present invention.

Fig. 1 shows a schematic presentation of the apparatus **1.** As shown, the apparatus **1** comprises an elongated main body **2,** an anchoring region **3,** a medical device attachment region **4,** and a connection region **5.**

Still referring to Fig. 1, the elongated main body **2** has a main body proximal end **6** and a main body distal end **7.** The elongated main body **2** is not limited to a particular elongated size or configuration. In some embodiments, the elongated main body **2** has a length less that is extendable to approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.) and retractable to approximately 0.2 meters (e.g., 0.01 meters, 0.05, 0.1, 0.15, 0.2, 0.3, 0.35, 0.4, 0.48, 0.5, 0.51, 0.55, 0.6, 1, 1.5, etc.). In some embodiments, the elongated main body **2** has a width that is between approximately 0.005 meters and 0.3 meters (e.g., between 0.01 and 0.5 meters, 0.015 and 0.4, 0.02 and 0.3, etc.). The elongated main body **2** is not limited to a particular weight. In some embodiments, the weight of the elongated main body **2** is such that it does not compromise the integrity of any function or purpose of the apparatus **1.**

The elongated main body **2** is not limited to a particular manner for extending or retracting its length. In some embodiments, the elongated main body **2** utilizes, for example, a telescoping hydraulic system to expand or retract its length. In some embodiments, the elongated main body **2** is configured to lock its length at any desired distance.

The elongated main body **2** is not limited to a particular material composition. Indeed, those of skill in the art would readily know of an applicable type of material composition for the elongated main body **2.** In some embodiments, the elongated main body **2** is made of any suitable metal (e.g., stainless steel, titanium, niobium, tantalum, nitinol, copper, and/or a mixture thereof). In some embodiments, the elongated main body **2** is made of plastic. In some embodiments, the elongated main body **2** is made of a mixture of metal and plastic. In some embodiments, the material composition of the elongated main body **2** is such that it does not compromise the integrity of any function or purpose of the apparatus **1.**

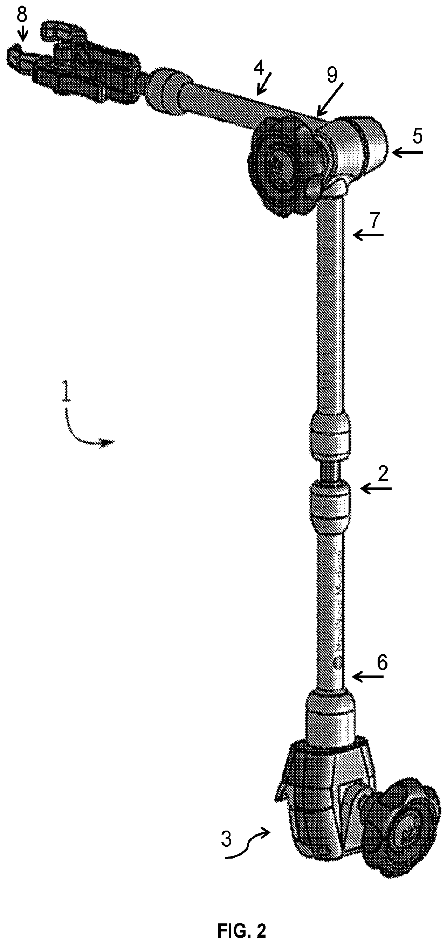

Fig. 2 shows drawing of an apparatus **1,** having an elongated main body **2,** an anchoring region **3,** a medical device attachment region **4,** and a connection region **5.** As can be seen, the elongated main body **2** has therein a main body proximal end **6** and a main body distal end **7.** Furthermore as can be seen, the length of the elongated main body **2** is greater than its width. In addition, within this embodiment, the elongated main body **2** is divided at its mid-region thereby facilitating the expanding or retracting of its length (e.g., via a hydraulic telescoping design).

Referring again to Fig. 1, the anchoring region **3** is shown engaged with the main body proximal end **6.** The anchoring region **3** is not limited to particular manner of engaging with the main body proximal end **6.** In some embodiments, the engagement between the anchoring region **3** and the main body proximal end **6** is such that it does not compromise the integrity of any function or purpose of the apparatus **1.**

The anchoring region **3** is configured such that it permits the apparatus **1** as a whole to attach onto or with an anchorable region. The anchoring region **3** is not limited to attaching onto or with a particular type of anchorable region. Examples of anchorable regions for which the anchoring region **3** is able to attach onto or with include, but are not limited to, a medical procedure table or tray, a medical procedure device able to bear the weight of the apparatus **1,** a patient chair or bed, railing on a patient operating table, etc. In some embodiments, the anchorable region is any type of region for which the anchoring element **3** can attach onto or with such that it does not compromise the integrity of any function or purpose of the apparatus **1.**

In some embodiments, following attachment onto or with an anchorable region, the anchoring region **3** is able to lock such attachment such that absent unlocking the anchoring region **3** is immobilized. The anchoring region is not limited to a particular manner of locking the anchoring region **3** following attachment onto or with an anchorable region.

In some embodiments, the anchoring region attaches onto or with an anchorable region through a fixed / movable mechanism. In some embodiments, the anchoring region attaches onto or with an anchorable region through a compression based mechanism. For example, in some embodiments, the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases in size with decreased amounts of compression. Indeed, in some embodiments, the compression based mechanism resembles a clamping mechanism wherein tension is applied to the clamp thereby decreasing the size of the clamping region which thereby permits a tight adherence between the anchoring region and the anchorable region.

Still referring to Fig. 1, the anchoring region **3** is not limited to a particular size or configuration. In some embodiments, the size or configuration of the anchoring region **3** is such that it is able to attach onto or with an anchorable region region whereby such attachment is sufficient to immobilize the anchoring region **3.** In some embodiments, the size or configuration of the anchoring region **3** is such that it is able to immobilize the anchoring region **3** such that it does not compromise the integrity of any function or purpose of the apparatus **1.** In some embodiments, the width and height of the anchoring region **3** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The anchoring region **3** is not limited to a particular weight. In some embodiments, the weight of the anchoring region **3** is such that it does not compromise the integrity of any function or purpose of the apparatus **1.**

The anchoring region **3** is not limited to a particular material composition. Indeed, those of skill in the art would readily know of an applicable type of material composition for the anchoring region **3.** In some embodiments, the anchoring region **3** is made of any suitable metal (e.g., stainless steel, titanium, niobium, tantalum, nitinol, copper, and/or a mixture thereof). In some embodiments, the anchoring region **3** is made of plastic. In some embodiments, the anchoring region **3** is made of a mixture of metal and plastic. In some embodiments, the material composition of the anchoring region **3** is such that it does not compromise the integrity of any function or purpose of the apparatus **1.**

Referring to Fig. 2, the anchoring element **3** is shown engaged with the main body proximal end **6.** As can be seen, within this embodiment, the anchoring element **3** has thereon a fastening knob which can be used to increase compression within a compressible region thereby facilitating attachment of the anchoring region **3** onto or with an anchorable region.

Referring again to Fig. 1, a medical device attachment region **4** having a medical device attachment region distal end **8** and a medical device attachment region proximal end **9** is shown. In addition, as can be seen, the medical device attachment region proximal end **9** is shown engaged with the connection region **5.**

The medical device attachment region proximal end **9** is not limited to a particular elongated size or configuration. In some embodiments, as can be seen from Fig. 1, the medical device attachment region proximal end **9** is elongated. In some embodiments, the medical device attachment region proximal end **9** has a length less that is extendable to approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.) and retractable to approximately 0.5 meters (e.g., 0.1 meters, 0.2, 0.3, 0.35, 0.4, 0.48, 0.5, 0.51, 0.55, 0.6, 1, 1.5, etc.). In some embodiments, the medical device attachment region proximal end **9** has a width that is between approximately 0.025 meters and 0.3 meters (e.g., between 0.01 and 0.5 meters, 0.015 and 0.4, 0.02 and 0.3, etc.). The medical device attachment region proximal end 9 is not limited to a particular weight. In some embodiments, the weight of the medical device attachment region proximal end **9** is such that it does not compromise the integrity of any function or purpose of the apparatus **1.**

The medical device attachment region proximal end **9** is not limited to a particular manner for extending or retracting its length. In some embodiments, the medical device attachment region proximal end **9** utilizes, for example, a telescoping hydraulic system to expand or retract its length. In some embodiments, the medical device attachment region proximal end **9** is configured to lock its length at any desired distance. In some embodiments, the medical device attachment region proximal end **9** is flexible such that it can be bent or shaped in any desired manner (e.g., a goose-neck formation).

The medical device attachment region proximal end **9** is not limited to a particular material composition. Indeed, those of skill in the art would readily know of an applicable type of material composition for the medical device attachment region proximal end **9.** In some embodiments, the medical device attachment region proximal end **9** is made of any suitable metal (e.g., stainless steel, titanium, niobium, tantalum, nitinol, copper, and/or a mixture thereof). In some embodiments, the medical device attachment region proximal end **9** is made of plastic. In some embodiments, the medical device attachment region proximal end **9** is made of a mixture of metal and plastic. In some embodiments, the medical device attachment region proximal end **9** is made of a flexible material which permits the region to be shaped in any desired manner. In some embodiments, the material composition of the medical device attachment region proximal end **9** is such that it does not compromise the integrity of any function or purpose of the apparatus **1.**

Referring to Fig. 2, the medical device attachment region proximal end **9** is shown engaged with the connection region **5** and the medical device attachment region distal end **9.** Furthermore as can be seen, the length of the medical device attachment region proximal end **9** is greater than its width.

Referring again to Fig. 1, the medical device attachment region distal end **8** is shown engaged with the medical device attachment region proximal end **9.** The medical device attachment region distal end **8** is not limited to particular manner of engaging with the medical device attachment region proximal end **9.** In some embodiments, the engagement between the medical device attachment region distal end **8** and the medical device attachment region proximal end **9** is such that it does not compromise the integrity of any function or purpose of the apparatus **1.**

The medical device attachment region distal end **8** is configured such that it permits a medical device to secure with the medical device attachment region distal end **8** and, as such, the apparatus **1** as a whole. The medical device attachment region distal end **8** is not limited to securing a particular type of medical device (see below for more detail).

In some embodiments, following securing of a medical device with the medical device attachment region distal end **8,** the medical device attachment region distal end **8** is able to lock such securing such that absent unlocking the medical device is immobilized with the medical device attachment region distal end **8.** The medical device attachment region distal end **8** is not limited to a particular manner of locking a medical device following securing with the medical device attachment region distal end **8.**

In some embodiments, the medical device attachment region distal end utilizes a torsion based mechanism for securing and locking a medical device. For example, in some embodiments, the medical device attachment region distal end has a mechanism that naturally rests in a locked position. In such embodiments, the torsion based mechanism is configured to be opened (e.g., manual opening) to a certain dimension such that it can accommodate a medical device (e.g., for purposes of securing the medical device within the medical device attachment region distal end). In such embodiments, following opening of the torsion based mechanism a release of such opening results in a closing of the mechanism (e.g., torsion based closing). In some embodiments, following positioning of a medical device within the medical device attachment region distal end such, closing of the mechanism results in a torsion based securing. In some embodiments, the torsion based mechanism a spring based. In some embodiments, the torsion based mechanism resembles an alligator clamp or clip.

In some embodiments, the medical device attachment region distal end utilizes a magnet based mechanism for securing and locking a medical device.

In some embodiments, the medical device attachment region distal end utilizes a compression based mechanism for securing and locking a medical device. For example, in some embodiments, the medical device attachment region distal end has a medical device attachment region distal end compressible region and a medical device attachment region distal end compressor region, wherein the medical device attachment region distal end compressor region is configured to apply a range of compression to the medical device attachment region distal end compressible region such that the size of the medical device attachment region distal end compressible region decreases with increased amounts of compression and increases in size with decreased amounts of compression. Indeed, in some embodiments, the compression based mechanism resembles a clamping mechanism wherein tension is applied to the clamp thereby decreasing the size of the clamping region which thereby permits a tight securing of a medical device within the medical device attachment region distal end.

In some embodiments, the mechanism within the medical device attachment region distal end for securing and locking a medical device is configured such that it does not obscure and/or hinder access to a secured medical device. For example, in some embodiments, the mechanism within the medical device attachment region distal end for securing and locking a medical device is sized such that a user can manipulate and/or read a medical device that is secured therein. In some embodiments, the mechanism within the medical device attachment region distal end for securing and locking a medical device is no larger than one or two adult human fingers.

Still referring to Fig. 1, the medical device attachment region distal end **8** is not limited to a particular size or configuration. In some embodiments, the size or configuration of the medical device attachment region distal end **8** is such that it is able to secure any type of medical device having any kind of size. In some embodiments, the size or configuration of the medical device attachment region distal end **8** is such that it is able to secure a medical device without compromising the integrity of any function or purpose of the apparatus **1.** In some embodiments, the width and height of the medical device attachment region distal end **8** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The medical device attachment region distal end **8** is not limited to a particular weight. In some embodiments, the weight of the medical device attachment region distal end **8** is such that it does not compromise the integrity of any function or purpose of the apparatus **1.**

The medical device attachment region distal end **8** is not limited to a particular material composition. Indeed, those of skill in the art would readily know of an applicable type of material composition for the medical device attachment region distal end **8.** In some embodiments, the medical device attachment region distal end **8** is made of any suitable metal (e.g., stainless steel, titanium, niobium, tantalum, nitinol, copper, and/or a mixture thereof). In some embodiments, the medical device attachment region distal end **8** is made of plastic. In some embodiments, the medical device attachment region distal end **8** is made of a mixture of metal and plastic. In some embodiments, the material composition of the medical device attachment region distal end **8** is such that it does not compromise the integrity of any function or purpose of the apparatus **1.**

Referring to Fig. 2, the medical device attachment region distal end **8** is shown engaged with the medical device attachment region proximal end **9.** As can be seen, within this embodiment, the medical device attachment region distal end **8** has thereon a dual prong that is able to be increased or decreased in size for purposes of applying or relieving a compression force onto a medical device (e.g., for purposes of securing or releasing a secured medical device with or from the medical device attachment region distal end **8).**

In some embodiments, the medical device attachment region further comprises one or more extensions for securing additional medical devices (e.g., additional energy delivery devices, light emitting objects, any other medical device). Such embodiments are not limited to a particular type or kind of extension for securing additional medical devices. In some embodiments, the extension is elongated with an additional device securing feature. In some embodiments, such an extension can be expanded or retracted to any desirable length. In some embodiments, the one or more extensions attach with the medical device attachment region via a bolting mechanism.

Referring again to Fig. 1, the connection region **3** is shown engaged with the medical device attachment region distal end **9** and the main body distal end **7.** The connection region **3** is not limited to particular manner of engaging with the medical device attachment region distal end **9** and the main body distal end **7.** In some embodiments, the engagement between the connection region **3** and the medical device attachment region distal end **9** and the main body distal end **7** is such that it does not compromise the integrity of any function or purpose of the apparatus **1.**

In some embodiments, the engagement between the connection region **3** and main body distal end **7** is such that it immobilizes the elongated main body **2** in a fixed position.

In some embodiments, the engagement between the connection region **3** and the medical device attachment region distal end **9** is such that it renders the medical device attachment region **4** capable of projection therefrom in any desired manner. For example, in some embodiments, the engagement between the connection region **3** and the medical device attachment region distal end **9** is such that the medical device attachment region **4** can be positioned in any projection therefrom (e.g., a radial projection from 0° to 360°). In some embodiments, the engagement between the connection region **3** and the medical device attachment region distal end **9** is such that the medical device attachment region **4** can be rotated about the axis of the connection region **3** (e.g., a rotational projection from 0° to 360°). Indeed, in some embodiments, the engagement between the connection region **3** and the medical device attachment region distal end **9** is such that the medical device attachment region **4** can be articulated in any desired manner.

In some embodiments, the connection region **3** is configured to lock the medical device attachment region **4** in a fixed projection. The connection region **3** is not limited to a particular manner of locking the medical device attachment region **4** in a fixed projection. In some embodiments, the connection region **3** has utilizes a tension applicator based mechanism for locking and unlocking the medical device attachment region **4** in a fixed projection.

Still referring to Fig. 1, the connection region **3** is not limited to a particular size or configuration. In some embodiments, the size or configuration of the connection region **3** is such that it is able to engage the medical device attachment region distal end **9** and the main body distal end **7** without compromising the integrity of any function or purpose of the apparatus **1.** In some embodiments, the size or configuration of the connection region **3** is such that it is able to engage the main body distal end **7** such that it immobilizes the elongated main body **2** in a fixed position. In some embodiments, the size or configuration of the connection region **3** is such that it renders the medical device attachment region **4** capable of projection therefrom in any desired manner.

In some embodiments, the width and height of the connection region **3** are independently between approximately 0.025 meters (e.g., between 0.01 and 0.5 meters, 0.015 and 0.4, 0.02 and 0.3, etc.) and approximately 2 meters (e.g., 1.5 meters, 1.6, 1.7, 1.75, 1.8, 1.9, 1.95, 1.99, 2.0, 2.01, 2.03, 2.5, 3, 5, 10, 15, etc.). The connection region **3** is not limited to a particular weight. In some embodiments, the weight of the connection region **3** is such that it does not compromise the integrity of any function or purpose of the apparatus **1.**

The connection region **3** is not limited to a particular material composition. Indeed, those of skill in the art would readily know of an applicable type of material composition for the connection region **3.** In some embodiments, the connection region **3** is made of any suitable metal (e.g., stainless steel, titanium, niobium, tantalum, nitinol, copper, and/or a mixture thereof). In some embodiments, the connection region **3** is made of plastic. In some embodiments, the connection region **3** is made of a mixture of metal and plastic. In some embodiments, the material composition of the connection region **3** is such that it does not compromise the integrity of any function or purpose of the apparatus **1.**

Referring to Fig. 2, the connection region **3** is shown engaged with the medical device attachment region distal end **9** and the main body distal end **7.** As can be seen, the engagement between the connection region **3** and the main body distal end **7** is such that it immobilizes the elongated main body **2** in a fixed position. As can be seen, the engagement between the connection region **3** and the medical device attachment region distal end **9** is such that the medical device attachment region **4** is projecting therefrom at approximately a 90° angle. Furthermore, as can be seen, the connection region **3** has thereon a fastening knob which can be used to increase or decrease tension onto the connection region **3** thereby locking or unlocking the medical device attachment region **4** in or from a desired projection.

The apparatuses of the present invention are not limited to securing a particular type of medical device. Indeed, the apparatuses of the present invention can be configured to secure any type or size of medical device.

In some embodiments, the medical device is an energy delivery device. The present invention is not limited to securing a particular type or kind of energy delivery device. Indeed, such embodiments contemplate the use of any type of device configured to deliver (e.g., emit) energy (e.g., ablation device, surgical device, etc.) (see, e.g., U.S. Patent Nos. 7,101,369, 7,033,352, 6,893,436, 6,878,147, 6,823,218, 6,817,999, 6,635,055, 6,471,696, 6,383,182, 6,312,427, 6,287,302, 6,277,113, 6,251,128, 6,245,062, 6,026,331, 6,016,811, 5,810,803, 5,800,494, 5,788,692, 5,405,346, 4,494,539, U.S. Patent Application Serial Nos. 11/728,460, 11/728,457, 11/728,428, 11/237,136, 11/236,985, 10/980,699, 10/961,994, 10/961,761, 10/834,802, 10/370,179, 09/847,181; Great Britain Patent Application Nos. 2,406,521, 2,388,039; European Patent No. 1395190; and International Patent Application Nos. WO 06/008481, WO 06/002943, WO 05/034783, WO 04/112628, WO 04/033039, WO 04/026122, WO 03/088858, WO 03/039385 WO 95/04385; each herein incorporated by reference in their entireties). Such devices include any and all medical, veterinary, and research applications devices configured for energy emission, as well as devices used in agricultural settings, manufacturing settings, mechanical settings, or any other application where energy is to be delivered.

In some embodiments, the apparatuses of the present invention are configured to secure energy delivery devices having therein antennae configured to emit energy (e.g., microwave energy, radiofrequency energy, radiation energy). The apparatuses are not limited to secure energy delivery devices having particular types or designs of antennae (e.g., ablation device, surgical device, etc.). In some embodiments, the apparatuses are configured to secure energy delivery devices having linearly shaped antennae (see, e.g., U.S. Patent Nos. 6,878,147, 4,494,539, U.S. Patent Application Serial Nos. 11/728,460, 11/728,457, 11/728,428, 10/961,994, 10/961,761; and International Patent Application No., WO 03/039385; each herein incorporated by reference in their entireties). In some embodiments, the apparatuses are configured to secure energy delivery devices having non-linearly shaped antennae (see, e.g., U.S. Patent Nos. 6,251,128, 6,016,811, and 5,800,494, U.S. Patent Application Serial No. 09/847,181, and International Patent Application No. WO 03/088858; each herein incorporated by reference in their entireties). In some embodiments, the apparatuses are configured to secure energy delivery devices having antennae with horn reflection components (see, e.g., U.S. Patent Nos. 6,527,768, 6,287,302; each herein incorporated by reference in their entireties). In some embodiments, the apparatuses are configured to secure energy delivery devices having antennae with a directional reflection shield (see, e.g., U.S. Patent No. 6,312,427; herein incorporated by reference in its entirety).

In some embodiments, the apparatuses are configured to secure energy delivery devices comprise coaxial transmission lines. Such devices are not limited to particular configurations of coaxial transmission lines. Examples of coaxial transmission lines include, but are not limited to, coaxial transmission lines developed by Pasternack, Micro-coax, and SRC Cables. In some embodiments, the coaxial transmission line has a center conductor, a dielectric element, and an outer conductor (e.g., outer shield). In some embodiments, the energy delivery devices comprise flexible coaxial transmission lines (e.g., for purposes of positioning around, for example, pulmonary veins or through tubular structures) (see, e.g., U.S. Patent Nos. 7,033,352, 6,893,436, 6,817,999, 6,251,128, 5,810,803, 5,800,494; each herein incorporated by reference in their entireties). In some embodiments, the energy delivery devices utilize antennae having rigid coaxial transmission lines (see, e.g., U.S. Patent No. 6,878,147, U.S. Patent Application Serial Nos. 10/961,994, 10/961,761, and International Patent Application No. WO 03/039385; each herein incorporated by reference in their entireties).

In some embodiments, the energy delivery devices have a coaxial transmission line positioned within the antenna, and a coaxial transmission line connecting with the antenna. In some embodiments, the size of the coaxial transmission line within the antenna is larger than the coaxial transmission line connected with the antenna. The coaxial transmission line within the antenna and the coaxial transmission line connecting with the antenna are not limited to particular sizes. For example, in some embodiments, whereas the coaxial transmission line connected with the antenna is approximately 0.032 inches, the size of the coaxial transmission line within the antenna is larger than 0.032 inches (e.g., 0.05 inches, 0.075 inches, 0.1 inches, 0.5 inches). In some embodiments, the coaxial transmission line within the antenna has an inner conductor that is stiff and thick. In some embodiments, the end of the coaxial transmission line within the antenna is sharpened for percutaneous use. In some embodiments, the dielectric coating of the coaxial transmission line within the antenna is PTFE (e.g., for purposes of smoothing transitions from a cannula to an inner conductor (e.g., a thin and sharp inner conductor)). In some embodiments, the shape of the coaxial transmission line and/or the dielectric element is selected and/or adjustable to fit a particular need.

In some embodiments, the energy delivery devices have a triaxial transmission line. In some embodiments, the apparatuses are configured to secure energy delivery devices having a triaxial microwave probe design where the outer conductor allows improved tuning of the antenna to reduce reflected energy through the transmission line. This improved tuning reduces heating of the transmission line allowing more power to be applied to the tissue and/or a smaller transmission line (e.g. narrower) to be used. Further, the outer conductor may slide with respect to the inner conductors to permit adjustment of the tuning to correct for effects of the tissue on the tuning. In some embodiments, and outer conductor is stationary with respect to the inner conductors. In some embodiments, the apparatuses are configured to secure energy delivery devices having antennae with a probe having a first conductor and a tubular second conductor coaxially around the first conductor but insulated therefrom (e.g. insulated by a dielectric material and/or coolant). A tubular third conductor is fit coaxially around the first and second conductors. The first conductor may extend beyond the second conductor into tissue when a proximal end of the probe is inserted into a body. The second conductor may extend beyond the third conductor into the tissue to provide improved tuning of the probe limiting power dissipated in the probe outside of the exposed portions of the first and second conductors. The third tubular conductor may be a channel catheter for insertion into the body or may be separate from a channel catheter. In some embodiments, the apparatuses are configured to secure energy delivery devices comprising first, second, and third conductors sufficiently flexible to navigate a winding path (e.g. through a branched structure within a subject (e.g. through the brachial tree)). In some embodiments, the first and second conductors may fit slidably within the third conductor. In some embodiments, the apparatuses are configured to secure energy delivery devices having a probe that facilitates tuning of the probe in tissue by sliding the first and second conductors inside of the third conductor. In some embodiments, the probe includes a lock attached to the third conductor to adjustably lock a sliding location of the first and second conductors with respect to the third conductor. In some embodiments, the apparatuses are configured to secure energy delivery devices having a triaxial transmission line, as described in U.S. Pat. No. 7,101,369, U.S. Pat. App. No. 2007/0016180, U.S. Pat. App. No.2008/0033424, U.S. Pat. App. No. 20100045558, U.S. Pat. App. No. 20100045559, herein incorporated by reference in their entireties.

In certain embodiments, the present invention provides methods for securing a medical device in a desire position, comprising securing an apparatus of the invention to an anchorable region, securing a medical device with the medical device attachment region distal end, and adjusting the apparatus such that the secured medical device is in a desired position, wherein the adjusting comprises adjusting one or more of the following: adjusting the projection of the medical device attachment region from the connection region, adjusting the length of the medical device attachment region proximal end, and adjusting the length of the elongated main body.

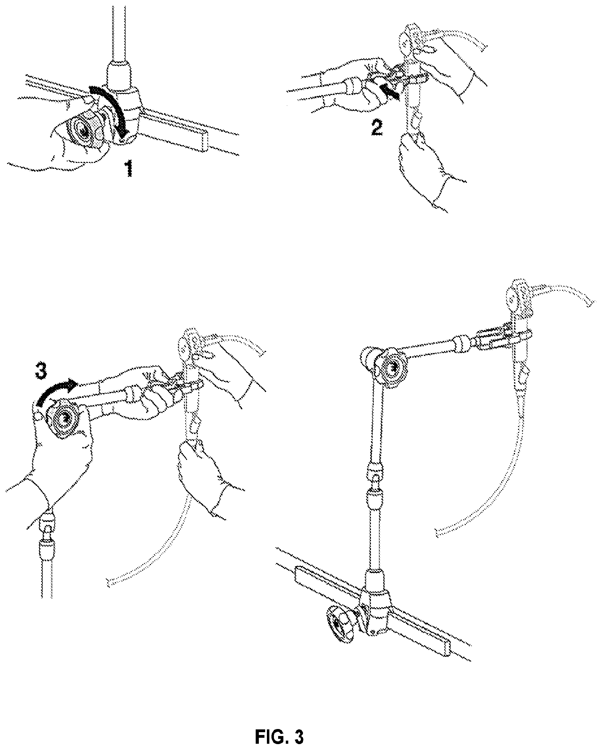

Indeed, Fig. 3 shows a typical mounting procedure with an apparatus of the present invention and the securing of a medical device with the apparatus. As shown in "1", the anchoring region of the apparatus is attached with an anchorable region. As shown, the anchoring region is positioned such that upon rotation of the rotational element a compressional force is applied thereby resulting in attachment of the apparatus onto the anchorable region. Next, as shown in "2", a medical device is secured within the medical device attachment region. Next, as shown in "3", the projection of the medical device attachment region from connection region is adjusted to a desired projection and locked into place.

The apparatuses of the present invention can be used, as such, within any type of medical procedure involving the use of a medical device. Indeed, such apparatuses greatly improve the efficiency of such medical procedures through fixing the medical device into a desired position and thereby removing the necessity of physically holding such a medical device at a desired location (e.g., by a physician, nurse, assistant, etc.).

In some embodiments, the apparatuses of the present invention are configured for use with systems comprising, for example, a primary catheter, wherein the primary catheter comprises a hollow primary lumen; a channel catheter, wherein the channel catheter is concentrically positioned within the hollow primary lumen, and wherein the channel catheter comprises a channel lumen; a steerable navigation catheter, wherein the steerable navigation catheter is configured to fit within the channel lumen, and wherein the steerable navigation catheter comprises a steerable tip and position sensing element; and a microwave energy delivery device, wherein the energy delivery device is configured to fit within the channel lumen, and wherein the microwave energy delivery device comprises, within the channel lumen, first, second and third conductors therein.

In some embodiments, any suitable endoscope or bronchoscope known to those in the art finds use as a primary catheter of such a system. In some embodiments, a primary catheter adopts characteristics of one or more endoscopes and/or bronchoscopes known in the art, as well as characteristics described herein. One type of conventional flexible bronchoscope is described in U.S. Pat. No. 4,880,015, herein incorporated by reference in its entirety. The bronchoscope measures 790 mm in length and has two main parts, a working head and an insertion tube. The working head contains an eyepiece; an ocular lens with a diopter adjusting ring; attachments for suction tubing, a suction valve, and light source; and an access port or biopsy inlet, through which various devices and fluids can be passed into the working channel and out the distal end of the bronchoscope. The working head is attached to the insertion tube, which typically measures 580 mm in length and 6.3 mm in diameter. The insertion tube contains fiberoptic bundles, which terminate in the objective lens at the distal tip, light guides, and a working channel. Other endoscopes and bronchoscopes which may find use in such systems, or portions of which may find use with the present invention, are described in U.S. Pat. No. 7,473,219; U.S. Pat. No. 6,086,529; U.S. Pat. No. 4,586,491; U.S. Pat. No. 7,263,997; U.S. Pat. No. 7,233,820; and U.S. Pat. No. 6,174,307.

In some embodiments, the systems provide a channel catheter (a.k.a. guide catheter, sheath, sheath catheter, etc.). In some embodiments, a guide catheter is configured to fit within the lumen of a primary catheter and contains a channel lumen of sufficient diameter (e.g. 1 mm .. 2 mm... 3 mm... 4 mm .. 5 mm) to accommodate a steerable navigation catheter and/or one or more suitable tools (e.g. energy delivery device). In some embodiments, a channel catheter is of sufficient length to extend from an insertion site (e.g. mouth, incision into body of subject, etc.) through the trachea and/or bronchial tree to a treatment site in the peripheral lung (e.g. 50 cm... 75 cm... 1 m... 1.5 m... 2m). In some embodiments, a channel catheter is of sufficient length to extend beyond the reach of a primary catheter to reach a treatment site (e.g. peripheral lung tissue). In some embodiments, a channel catheter is highly flexible to access a circuitous route through a subject (e.g. through a branched structure, through the bronchial tree, etc.). In some embodiments, a channel catheter is constructed of braided material to provide both strength and flexibility, as is understood in the art. In some embodiments, a channel catheter comprises the outer conductor of a triaxial transmission line. In some embodiments, a channel catheter comprises a navigation and/or steering mechanism. In some embodiments, a channel catheter is without an independent means of navigation, position recognition, or maneuvering. In some embodiments, a channel catheter relies upon the primary catheter or steerable navigation catheter for placement.

In some embodiments, the systems provide a steerable navigation catheter. In some embodiments, a steerable navigation catheter is configured to fit within the lumen of a channel catheter. In some embodiments, a steerable navigation catheter has a similar diameter to energy transmission lines described herein (e.g. 0.2 mm .. 0.5 mm... 1.0 mm .. 1.5 mm .. 2.0mm). In some embodiments, a steerable navigation catheter is of sufficient length to extend from an insertion site (e.g. mouth, incision into body of subject, etc.) to a treatment site (e.g. through the trachea and/or bronchial tree to a treatment site in the peripheral lung (e.g. 50 cm... 75 cm... 1 m... 1.5 m... 2m). In some embodiments, a channel catheter is of sufficient length to extend beyond the reach of a primary catheter to reach a treatment site (e.g. peripheral lung tissue). In some embodiments, a steerable navigation catheter engages a channel catheter such that movement of the steerable navigation catheter results in synchronous movement of the channel catheter. In some embodiments, as a steerable navigation catheter is inserted along a path in a subject, the channel catheter surrounding the steerable navigation catheter moves with it. In some embodiments, a channel catheter is placed within a subject by a steerable navigation catheter. In some embodiments, a steerable navigation catheter can be disengaged from a channel catheter. In some embodiments, disengagement of a steerable navigation catheter and channel catheter allows movement of the steerable navigation catheter further along a pathway without movement of the channel catheter. In some embodiments, disengagement of a steerable navigation catheter and channel catheter allows retraction of the steerable navigation catheter through the channel catheter without movement of the channel catheter.

In some embodiments, all inserted components of a system or device are configured for movement along a narrow and circuitous path through a subject (e.g. through a branched structure, through the bronchial tree, etc.). In some embodiment, components comprise a flexible material configured for tight turning radiuses. In some embodiment, necessarily rigid components are reduced in size (e.g. short length) to allow for tight turning radiuses.

As such, in certain embodiments, the present invention provides methods for placing a microwave energy delivery device at a difficult to reach treatment site comprising providing an apparatus of the present invention and the above described system, wherein the steerable navigation catheter is within the channel lumen, and the channel catheter is concentrically positioned within the hollow primary lumen. In some embodiments, the methods involve securing the apparatus to an anchorable region; securing the microwave energy delivery device with the medical device attachment region distal end; adjusting the apparatus such that the secured microwave energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; inserting the primary catheter into an opening in a subject and directing the primary catheter towards the treatment site until further advance is constrained by the diameter of the primary catheter; advancing the channel catheter beyond the distal end of the primary catheter and extending the channel catheter to the treatment site; securing the distal end of the channel catheter at the treatment site; withdrawing the steerable navigation catheter through the channel lumen and out the proximal end of the channel catheter; inserting the microwave energy delivery device through the channel lumen until the distal end of the microwave energy delivery device reaches the treatment site.

In some embodiments, the difficult to reach treatment site comprises the periphery of the lung and/or a peripheral lung nodule. In some embodiments, the lung nodule is accessed through the bronchial tree.

In certain embodiments, the present invention provides methods for treating a peripheral lung tissue region comprising providing an apparatus of the present invention and a system as described above, wherein the steerable navigation catheter is within the channel lumen, and the channel catheter is concentrically positioned within the hollow primary lumen. In some embodiments, the methods involve securing the apparatus to an anchorable region; securing the energy delivery device with the medical device attachment region distal end; adjusting the apparatus such that the secured energy delivery device is in a desired position, wherein the adjusting comprises adjusting one or more of the following: adjusting the projection of the medical device attachment region from the connection region, adjusting the length of the medical device attachment region proximal end, and adjusting the length of the elongated main body; steering the energy delivery device through the subject's lung and positioning the microwave energy delivery device at a target peripheral lung tissue region, and ablating the target peripheral lung tissue region with energy from the microwave energy delivery device, wherein the steering is through the subject's mouth, through the subject's trachea, and through the subject's lung.

In certain embodiments, the present invention provides kits comprising an apparatus of the present invention and a system comprises one or more of a primary catheter, wherein the primary catheter comprises a hollow primary lumen; a channel catheter, wherein the channel catheter is concentrically positioned within the hollow primary lumen, and wherein the channel catheter comprises a channel lumen; a steerable navigation catheter, wherein the steerable navigation catheter is configured to fit within the channel lumen, and wherein the steerable navigation catheter comprises a steerable tip and position sensing element; and a microwave energy delivery device, wherein the energy delivery device is configured to fit within the channel lumen, and wherein the microwave energy delivery device comprises, within the channel lumen, first, second and third conductors therein. In some embodiments, the primary catheter comprises a bronchoscope. In some embodiments, the channel catheter comprises a braided material that provides flexibility. In some embodiments, the inner conductor is hollow. In some embodiments, the hollow of the inner conductor is in fluid communication with a coolant source. In some embodiments, the space between the inner and outer conductors comprises a dielectric material. In some embodiments, the space between the inner and outer conductors comprises air channels.

In some embodiments, the systems further comprise a handle for manipulation of one or more of the primary catheter, the channel catheter, the steerable navigation catheter, and the microwave energy delivery device.

In some embodiments, the systems further comprise a processor configured to operate power delivery to the microwave energy delivery device.

In some embodiments, the systems further comprise a microwave power supply in electrical communication with the microwave energy delivery device.

## Claims

1. An apparatus (1) for securing a medical device, comprising
an elongated main body (2) having a main body proximal end (6) and a main body distal end (7), wherein the length of the main body is greater than the width of the main body, wherein the length of the main body is expandable or retractable, wherein the main body has a length that is extendable to 2 meters and retractable to 0.2 meters, wherein the main body has a width that is between 0.005 meters and 0.3 meters,
an anchoring region (3) engaged with the main body proximal end, wherein the anchoring region is configured to lock onto an anchorable region, wherein upon locking with an anchorable region the anchoring region and elongated main body are locked into a fixed position,
a medical device attachment region (4) having a medical device attachment region distal end (8) and a medical device attachment region proximal end (9),
wherein the length of the medical device attachment region proximal end is expandable or retractable,
wherein the medical device attachment region proximal end is elongated such that the length of the medical device attachment region proximal end is greater than the width of the medical device attachment region proximal end,
wherein the medical device attachment region proximal end has a length that is extendable to 2 meters and retractable to 0.5 meters, wherein the medical device attachment region proximal end has a width that is between 0.025 meters and 0.3 meters,
wherein the medical device attachment region distal end is configured to secure a medical device, and
a connection region (5) engaging the main body distal end and the medical device attachment region proximal end, wherein the connection region is adjustable such that the medical device attachment region can lockably project in any direction therefrom.

2. The apparatus of Claim 1, wherein the anchoring region has an anchoring region compressible region and an anchoring region compressor region, wherein the anchoring region compressor region is configured to apply a range of compression to the anchoring region compressible region such that the size of the anchoring region compressible region decreases with increased amounts of compression and increases with decreased amounts of compression, wherein the amount of applied compression is lockable.

3. The apparatus of Claim 1, wherein the medical device attachment region distal end is configured with a torsion based securing mechanism, wherein the torsion based securing mechanism naturally rests in a closed manner, wherein the torsion based securing mechanism is configured to be opened for purposes of accommodating a medical device, wherein releasing the torsion based securing mechanism from an opened manner naturally results in torsion based closing of the torsion based securing mechanism.

4. The apparatus of Claim 1 , wherein the medical device attachment region has thereon one or more extensions projecting therefrom, wherein the one or more extensions are configured to secure one or more medical devices.

5. A method of securing a medical device in a desired position, comprising:
securing an apparatus (1) as provided in Claim 1 to an anchorable region,
securing a medical device with the medical device attachment region distal end (8), wherein the medical device is an energy delivery device configured to ablate tissue,
and adjusting the apparatus such that the secured medical device is in a desired position, wherein the adjusting comprises adjusting one or more of the following:
adjusting the projection of the medical device attachment region (4) from the connection region (5),
adjusting the length of the medical device attachment region proximal end (9), and
adjusting the length of the elongated main body (2).

6. A kit comprising an apparatus (1) of Claim 1 and a system, wherein the system comprises:
a primary catheter, wherein the primary catheter comprises a hollow primary lumen;
a channel catheter, wherein the channel catheter is concentrically positioned within the hollow primary lumen, and wherein the channel catheter comprises a channel lumen;
a steerable navigation catheter, wherein the steerable navigation catheter is configured to fit within the channel lumen, and wherein the steerable navigation catheter comprises a steerable tip and position sensing element; and
an energy delivery device, wherein the energy delivery device is configured to fit within the channel lumen, and wherein the energy delivery device comprises, within the channel lumen, first, second and third conductors therein,
wherein the energy delivery device is a microwave ablation device or a radiofrequency energy delivery device.

7. The kit of Claim 6, wherein the primary catheter comprises a bronchoscope.

8. The kit of Claim 6, further comprising a handle for manipulation of one or more of the primary catheter, the channel catheter, the steerable navigation catheter, and the microwave ablation device.

9. The kit of Claim 6, further comprising a processor configured to operate power delivery to the microwave ablation device.

10. The kit of Claim 6, wherein the microwave ablation device is capable of delivering microwave energy through the channel catheter.

11. The kit of Claim 6, wherein the microwave ablation device comprises a coolant channel and wherein the system comprises a coolant source in fluid communication with the coolant channel.

12. The kit of Claim 6, wherein the channel catheter comprises a braided material that provides flexibility.

13. The kit of Claim 6, further comprising a microwave power supply in electrical communication with the microwave ablation device.

14. The kit of Claim 6, wherein the inner conductor is hollow.

15. The kit of Claim 14, wherein the hollow of the inner conductor is in fluid communication with a coolant source.

## Patentansprüche

1. Einrichtung (1) zur Sicherung einer medizinischen Vorrichtung, umfassend:
einen länglichen Hauptkörper (2), der ein proximales Hauptkörperende (6) und ein distales Hauptkörperende (7) aufweist, wobei die Länge des Hauptkörpers größer als die Breite des Hauptkörpers ist, wobei die Länge des Hauptkörpers ausziehbar oder einziehbar ist, wobei der Hauptkörper eine Länge aufweist, die auf 2 Meter ausziehbar ist oder auf 0,2 Meter einziehbar ist, wobei der Hauptkörper eine Breite aufweist, die zwischen 0,005 Meter und 0,3 Meter beträgt,
eine Verankerungsregion (3), die mit dem proximalen Hauptkörperende in Eingriff ist, wobei die Verankerungsregion so ausgestaltet ist, dass sie sich an einer verankerbaren Region verriegelt, wobei die Verankerungsregion und der längliche Hauptkörper beim Verriegeln mit einer verankerbaren Region in einer festen Position verriegelt werden,
eine Anbringungsregion (4) für eine medizinische Vorrichtung, die ein distales Ende (8) der Anbringungsregion für die medizinische Vorrichtung und ein proximales Ende (9) der Anbringungsregion für die medizinische Vorrichtung aufweist,
wobei die Länge des proximalen Endes der Anbringungsregion für die medizinische Vorrichtung ausziehbar oder einziehbar ist,
wobei das proximale Ende der Anbringungsregion für die medizinische Vorrichtung derart länglich ist, dass die Länge des proximalen Endes der Anbringungsregion für die medizinische Vorrichtung größer als die Breite des proximalen Endes der Anbringungsregion für die medizinische Vorrichtung ist, wobei das proximale Ende der Anbringungsregion für die medizinische Vorrichtung eine Länge aufweist, die auf 2 Meter ausziehbar und auf 0,5 Meter einziehbar ist, wobei das proximale Ende der Anbringungsregion für die medizinische Vorrichtung eine Breite aufweist, die zwischen 0,025 Meter und 0,3 Meter beträgt,
wobei das distale Ende der Anbringungsregion für die medizinische Vorrichtung so ausgestaltet ist, dass es eine medizinische Vorrichtung sichert, und
eine Verbindungsregion (5), die das distale Hauptkörperende und das proximale Ende der Anbringungsregion für die medizinische Vorrichtung in Eingriff bringt, wobei die Verbindungsregion derart anpassbar ist, dass die Anbringungsregion für die medizinische Vorrichtung verriegelbar in irgendeiner Richtung davon vorspringen kann.

2. Einrichtung nach Anspruch 1, wobei die Verankerungsregion eine zusammendrückbare Region der Verankerungsregion und eine zusammendrückende Region der Verankerungsregion aufweist, wobei die zusammendrückende Region der Verankerungsregion so ausgestaltet ist, dass sie einen Zusammendrückbereich auf die zusammendrückbare Region der Verankerungsregion derart anwendet, dass die Größe der zusammendrückbaren Region der Verankerungsregion bei zunehmenden Zusammendrückbeträgen abnimmt und bei abnehmenden Zusammendrückbeträgen zunimmt, wobei der Betrag des angewandten Zusammendrückens verriegelbar ist.

3. Einrichtung nach Anspruch 1, wobei das distale Ende zur Anbringung der medizinischen Vorrichtung mit einem torsionsbasierten Sicherungsmechanismus ausgestaltet ist, wobei der torsionsbasierte Sicherungsmechanismus auf eine geschlossene Weise natürlich ruht, wobei der torsionsbasierte Sicherungsmechanismus so ausgestaltet ist, dass er zu Zwecken des Aufnehmens einer medizinischen Vorrichtung geöffnet wird, wobei das Freigeben des torsionsbasierten Sicherungsmechanismus von einer geöffneten Weise natürlich das torsionsbasierte Schließen des torsionsbasierten Sicherungsmechanismus ergibt.

4. Einrichtung nach Anspruch 1, wobei die Region zur Anbringung der medizinischen Vorrichtung eine oder mehrere Erweiterungen daran aufweist, die davon vorspringen, wobei die eine oder mehreren Erweiterungen so ausgestaltet sind, dass sie eine oder mehrere medizinische Vorrichtungen sichern.

5. Verfahren zum Sichern einer medizinischen Vorrichtung in einer gewünschten Position, umfassend:
Sichern einer Einrichtung (1) nach Anspruch 1 an einer verankerbaren Region,
Sichern einer medizinischen Vorrichtung mit dem distalen Ende (8) der Anbringungsregion für die medizinische Vorrichtung, wobei die medizinische Vorrichtung eine Energielieferungsvorrichtung ist, die so ausgestaltet ist, dass sie Gewebe ablatiert,
und Einstellen der Einrichtung, sodass die gesicherte medizinische Vorrichtung sich in einer gewünschten Position befindet, wobei das Anpassen das Anpassen von einem oder mehreren von Folgendem umfasst:
Anpassen des Vorsprungs der Anbringungsregion (4) für die medizinische Vorrichtung von der Verbindungsregion (5),
Anpassen der Länge des proximalen Endes (9) der Anbringungsregion für die medizinische Vorrichtung, und
Anpassen der Länge des länglichen Hauptkörpers (2).

6. Kit, das eine Einrichtung (1) nach Anspruch 1 und ein System umfasst, wobei das System umfasst:
einen Hauptkatheter, wobei der Hauptkatheter ein hohles Hauptlumen umfasst;
einen Kanalkatheter, wobei der Kanalkatheter konzentrisch innerhalb des hohlen Hauptlumens positioniert ist und wobei der Kanalkatheter ein Kanallumen umfasst;
einen lenkbaren Navigationskatheter, wobei der lenkbare Navigationskatheter so ausgestaltet ist, dass er in das Kanallumen passt, und wobei der lenkbare Navigationskatheter ein lenkbares Spitzen- und Positionsabtastelement umfasst; und
eine Energielieferungsvorrichtung, wobei die Energielieferungsvorrichtung so ausgestaltet ist, dass sie in das Kanallumen passt, und wobei die Energielieferungsvorrichtung innerhalb des Kanallumens einen ersten, einen zweiten und einen dritten Leiter darin umfasst,
wobei die Energielieferungsvorrichtung eine Mikrowellen-Ablationsvorrichtung oder eine Hochfrequenz-Energielieferungsvorrichtung ist.

7. Kit nach Anspruch 6, wobei der Hauptkatheter ein Bronchoskop umfasst.

8. Kit nach Anspruch 6, das ferner einen Griff zur Manipulation von einem oder mehreren von dem Hauptkatheter, dem Kanalkatheter, dem lenkbaren Navigationskatheter oder der Mikrowellen-Ablationsvorrichtung umfasst.

9. Kit nach Anspruch 6, das ferner einen Prozessor umfasst, der so ausgestaltet ist, dass er Leistungslieferung zu der Mikrowellen-Ablationsvorrichtung betreibt.

10. Kit nach Anspruch 6, wobei die Mikrowellen-Ablationsvorrichtung in der Lage ist, Mikrowellenenergie durch den Kanalkatheter zu liefern.

11. Kit nach Anspruch 6, wobei die Mikrowellen-Ablationsvorrichtung einen Kühlmittelkanal umfasst und wobei das System eine Kühlmittelquelle in Fluidverbindung mit dem Kühlmittelkanal umfasst.

12. Kit nach Anspruch 6, wobei der Kanalkatheter ein geflochtenes Material umfasst, das Biegsamkeit bereitstellt.

13. Kit nach Anspruch 6, das ferner eine Mikrowellen-Leistungsversorgung in elektrischer Verbindung mit der Mikrowellen-Ablationsvorrichtung umfasst.

14. Kit nach Anspruch 6, wobei der innere Leiter hohl ist.

15. Kit nach Anspruch 14, wobei der Hohlraum des inneren Leiters in Fluidverbindung mit einer Kühlmittelquelle ist.

## Revendications

1. Appareil (1) de maintien d'un dispositif médical, comprenant
un corps principal allongé (2) ayant une extrémité proximale de corps principal (6) et une extrémité distale de corps principal (7), dans lequel la longueur du corps principal est supérieure à la largeur du corps principal, dans lequel la longueur du corps principal est expansible ou rétractable, dans lequel le corps principal a une longueur qui est extensible jusqu'à 2 mètres et rétractable jusqu'à 0,2 mètre, dans lequel le corps principal a une largeur qui est comprise entre 0,005 mètre et 0,3 mètre,
une région d'ancrage (3) en prise avec l'extrémité proximale de corps principal, dans lequel la région d'ancrage est configurée pour se verrouiller sur une région ancrable, dans lequel lors du verrouillage avec une région ancrable la région d'ancrage et le corps principal allongé sont verrouillés dans une position fixe,
une région de fixation de dispositif médical (4) ayant une extrémité distale de région de fixation de dispositif médical (8) et une extrémité proximale de région de fixation de dispositif médical (9),
dans lequel la longueur de l'extrémité proximale de région de fixation de dispositif médical est expansible ou rétractable,
dans lequel l'extrémité proximale de région de fixation de dispositif médical est allongée de telle sorte que la longueur de l'extrémité proximale de région de fixation de dispositif médical soit supérieure à la largeur de l'extrémité proximale de région de fixation de dispositif médical,
dans lequel l'extrémité proximale de région de fixation de dispositif médical a une longueur qui est extensible jusqu'à 2 mètres et rétractable jusqu'à 0,5 mètre, dans lequel l'extrémité proximale de région de fixation de dispositif médical a une largeur qui est comprise entre 0,025 mètre et 0,3 mètre,
dans lequel l'extrémité distale de région de fixation de dispositif médical est configurée pour maintenir un dispositif médical, et
une région raccord (5) mettant en prise l'extrémité distale de corps principal et l'extrémité proximale de région de fixation de dispositif médical, dans lequel la région raccord est ajustable de manière à ce que la région de fixation de dispositif médical puisse se projeter de manière verrouillable dans toute direction à partir de celle-ci.

2. Appareil selon la revendication 1, dans lequel la région d'ancrage a une région compressible de région d'ancrage et une région compresseur de région d'ancrage, dans lequel la région compresseur de région d'ancrage est configurée pour appliquer une plage de compression à la région compressible de région d'ancrage de telle sorte que la taille de la région compressible de région d'ancrage diminue avec des quantités accrues de compression et augmente avec des quantités réduites de compression, dans lequel la quantité de compression appliquée est verrouillable.

3. Appareil selon la revendication 1, dans lequel l'extrémité distale de région de fixation de dispositif médical est configurée avec un mécanisme de maintien basé sur la torsion, dans lequel le mécanisme de maintien basé sur la torsion reste naturellement dans une condition fermée, dans lequel le mécanisme de maintien basé sur la torsion est configuré pour être ouvert à des fins de réception d'un dispositif médical, dans lequel la libération du mécanisme de maintien basé sur la torsion d'une condition ouverte résulte naturellement en la fermeture basée sur la torsion du mécanisme de maintien basé sur la torsion.

4. Appareil selon la revendication 1, dans lequel sur la région de fixation de dispositif médical se trouvent une ou plusieurs extensions faisant saillie à partir de là, dans lequel les une ou plusieurs extensions sont configurées pour maintenir un ou plusieurs dispositifs médicaux.

5. Procédé pour maintenir un dispositif médical dans une position souhaitée, comprenant :
le maintien d'un appareil (1) selon la revendication 1 à une région ancrable,
le maintien d'un dispositif médical avec l'extrémité distale de région de fixation de dispositif médical (8), dans lequel le dispositif médical est un dispositif de délivrance d'énergie configuré pour réséquer un tissu,
et l'ajustement de l'appareil de manière à ce que le dispositif médical maintenu se trouve dans une position souhaitée, dans lequel l'ajustement comprend l'ajustement d'un ou plusieurs des suivants :
ajustement de la saillie de la région de fixation de dispositif médical (4) à partir de la région raccord (5),
ajustement de la longueur de l'extrémité proximale de région de fixation de dispositif médical (9), et
ajustement de la longueur du corps principal allongé (2).

6. Kit comprenant un appareil (1) selon la revendication 1 et un système, dans lequel le système comprend :
un cathéter primaire, dans lequel le cathéter primaire comprend une lumière primaire creuse ;
un cathéter à canal, dans lequel le cathéter à canal est concentriquement positionné à l'intérieur de la lumière primaire creuse, et dans lequel le cathéter à canal comprend une lumière de canal ;
un cathéter de navigation orientable, dans lequel le cathéter de navigation orientable est configuré pour s'ajuster à l'intérieur de la lumière de canal, et dans lequel le cathéter de navigation orientable comprend une pointe orientable et un élément de détection de position ; et
un dispositif de délivrance d'énergie, dans lequel le dispositif de délivrance d'énergie est configuré pour s'ajuster à l'intérieur de la lumière de canal, et dans lequel le dispositif de délivrance d'énergie comprend, à l'intérieur de la lumière de canal, des premier, deuxième et troisième conducteurs,
dans lequel le dispositif de délivrance d'énergie est un dispositif d'ablation microonde ou un dispositif de délivrance d'énergie radiofréquence.

7. Kit selon la revendication 6, dans lequel le cathéter primaire comprend un bronchoscope.

8. Kit selon la revendication 6, comprenant en outre une poignée pour la manipulation d'un ou plusieurs du cathéter primaire, du cathéter à canal, du cathéter de navigation orientable, et du dispositif d'ablation microonde.

9. Kit selon la revendication 6, comprenant en outre un processeur configuré pour mettre en œuvre la délivrance d'énergie au dispositif d'ablation microonde.

10. Kit selon la revendication 6, dans lequel le dispositif d'ablation microonde est capable de délivrer l'énergie microonde par l'intermédiaire du cathéter à canal.

11. Kit selon la revendication 6, dans lequel le dispositif d'ablation microonde comprend un canal de caloporteur et dans lequel le système comprend une source de caloporteur en communication fluidique avec le canal de caloporteur.

12. Kit selon la revendication 6, dans lequel le cathéter à canal comprend un matériau tressé qui fournit une flexibilité.

13. Kit selon la revendication 6, comprenant en outre une source d'énergie microonde en communication électrique avec le dispositif d'ablation microonde.

14. Kit selon la revendication 6, dans lequel le conducteur interne est creux.

15. Kit selon la revendication 14, dans lequel le creux du conducteur interne est en communication fluidique avec une source de caloporteur.
